# EUROPEAN PATENT SPECIFICATION

(11) **EP 1 677 806 B1**
(45) Date of publication and mention of the grant of the patent: **19.11.2008**
(21) Application number: 04761743.6
(22) Date of filing: 27.08.2004
(51) Int. Cl.: A61K 31/728, A61P 19/04

(54) **METHODS FOR TREATING ACUTE AND OVERUSE SPRAIN AND STRAIN USING HYALURONIC ACID**
VERFAHREN ZUR BEHANDLUNG VON AKUTER UND DURCH ÜBERMÄSSIGE BEANSPRUCHUNG VERURSACHTER ZERRUNG UND VERSTAUCHUNG MIT HYALURONSÄURE
METHODES PERMETTANT DE TRAITER UNE ENTORSE ET UNE FOULURE AIGUES PROVOQUEES PAR LE SURMENAGE MUSCULAIRE AU MOYEN D'ACIDE HYALURONIQUE

(30) Priority: 09.10.2003 US 510684 P
(43) Date of publication of application: 12.07.2006
(73) Proprietor: Petrella, Robert John, London, Ontario N5X 3W7 (CA)
(72) Inventor: Petrella, Robert John, London, Ontario N5X 3W7 (CA)
(74) Representative: Harding, Charles Thomas
(86) International application number: PCT/CA2004/001579
(87) International publication number: WO 2005/032562

(56) References cited:
- US-A- 5 079 236
- NIXON A J: "USE OF HYALURONIC ACID FOR TENDON AND TENDON SHEATH DISORDERS IN THE HORSE" JOURNAL OF THE AMERICAN VETERINARY MEDICAL ASSOCIATION, vol. 194, no. 12, 1989, page 1779, XP009078917 & 126TH ANNUAL AMERICAN VETERINARY MEDICAL ASSOCIATION MEETING, ORLANDO, FLORIDA, USA, JULY 15-19, 198 ISSN: 0003-1488
- DATABASE MEDLINE [Online] US NATIONAL LIBRARY OF MEDICINE (NLM), BETHESDA, MD, US; June 1992 (1992-06), RAPP H J ET AL: "[Diagnosis and therapy of tendinitis exemplified by the athletic horse]" XP002420134 Database accession no. NLM1641754 & SPORTVERLETZUNG SPORTSCHADEN : ORGAN DER GESELLSCHAFT FÜR ORTHOPÄDISCH-TRAUMATOLOGISCHE SPORTMEDIZIN JUN 1992, vol. 6, no. 2, June 1992 (1992-06), pages 77-88, ISSN: 0932-0555
- ZATTONI ET AL.: 'Efficacy and tolerability of hyaluronic acid in acute knee injury: a controlled clinical study' EUR. J. RHEUMATOL. INFLAMM. vol. 15, no. 1, 1995, pages 63 - 69, XP009078880
- DEPARTEMENT OF HEALTH & HUMAN SERVICES: 'Food and drug administration, Hyalgan FDA #950027', [Online] 28 May 1997, pages 1 - 54, XP003010367 Retrieved from the Internet: <URL:http://www.fda.gov/cdrh/pdf/p950027.pd f>

## Description

### FIELD OF THE INVENTION

The present invention relates to the compositions comprising hyaluronic acid for the treatment of sprain and strain in animals and humans.

### BACKGROUND OF THE INVENTION

Every year, millions of people seek medical treatments for acute or overuse injury to ligaments (sprain), or musculo-tendinous structures (strain). Sprain can vary from first degree (slight ligamentous tear) to second degree (greater tearing with blood clot formation and moderate functional impairment) to third degree (total separation of the ligament associated with loss of function and mechanical stability). Symptoms include pain, heat, redness, swelling and functional loss. Therapies for sprain are directed at decreasing inflammation and pain. Treatment of mild to moderate sprains (first and second degrees) is usually done at home with rest, ice, compression and elevation (the so-called RICE treatment, rest-ice-compression-elevation), the use of nonsteroidal anti-inflammatory drugs (NSAIDs) that include aspirin, ibuprofen and naproxen, or immobilization with various devices including braces or plaster casts. More severe injury may require splinting, casting, or even surgical stabilization. Strain may result from a traumatic injury or from improper or overuse of a muscle-tendon unit characterized by pain, swelling and impaired movement when using the injured muscles. Treatment includes cold or heat compresses, immobilization, and/or the use of NSAIDs. Sprains and strains can affect any ligamentous or muscle-tendon structure and include, but are not limited to the ligaments and tendons associated with the following joints and structures: foot, plantar fascia, ankle, knee, patellar-femoral structure, hip, ilio-tibial band, back, shoulder, elbow, wrist, hand, jaw, and neck.

Hyaluronic acid (hereinafter, "HA"), also known as hyaluronan, hyaluronate or sodium hyaluronate, is an abundant non-sulfated glycosaminoglycan that is present in all joint tissues. HA is a naturally occurring linear polysaccharide composed of β-1,4-linked D-glucuronic acid-(β-1,3)-N-acetyl-D-glucosamine dissacharide units. In its native form, HA exists as a high molecular weight polymer (about 10⁶-10⁷ Da). In normal human synovial fluid, the molecular weight of HA is between about 6-7 x 10⁶ Da, and the concentration is about 2-4 mg/ml. HA synthesized by synoviocytes is responsible for the viscoelastic properties of synovial fluid and plays a fundamental role in the maintenance of the trophic status of the cartilage. In joint disease there is a reduction in both the concentration and molecular weight of HA. Intra-articular injection of exogenous high molecular weight HA (> 5 x 10⁶ Da) was found to improve function in humans with osteoarthritis or rheumatoid arthritis (Maheu et al., Int. J. Clin. Pract. 56:804-813, 2002; Matsuno et al., Inflamm. Res. 48:154-159, 1999). Three to five weekly intra-articular injections were required to significantly improve the pain and the functional status of patients with osteoarthritis, the effect lasting at least six months and up to one year after treatment cessation (Maheu et al., Int. J. Clin. Pract. 56:804-813, 2002). It was believed that intra-articular administration of HA may reverse HA degradation observed in osteoarthritis and to restore synovial fluid viscosity (viscosupplementation) (Balazs and Denlinger, J. Rheumatol. 20:3-9, 1993). Intra-articular administration of HA was also found to improve function in humans with acute knee injury (Zattono et al., Eur, J. Rheumatol. Inflamm. 15:53-69, 1995). While intra-articular administration of HA is has been proposed for various conditions, it is a complex process as locating the joint cavity during an intra-articular procedure is relatively difficult. Improper injection may lead to a variety of complications.

Most known medical therapies for sprain and strain, whether directed to decreasing inflammation and/or pain, have proven to be less than adequate. Many of these therapies have limited efficacy or have significant side effects.

There is a continuing need for novel agents and methods for treating sprain and strain.

US 5 079 236 relates to pure, sterile, pyrogen-free hyaluronic acid formulations their methods of preparation and methods of use.

Nixon A J, Journal of the American Veterinary Medical Association, Vol. 194, No. 12, 1989, page 1779 relates to use of hyaluronic acid for tendon and tendon sheath disorders in the horse.

Rapp, H. J. et al., Medline accession no. NLM1641754 relates to diagnosis and therapy of tendonitis exemplified by the athletic horse.

Zattoni et al. Eur. J. Rheumatol. Inflamm. Vol. 15, 1995, pages 63-69 relates to efficacy and tolerability of hyaluronic acid in acute knee injury:a controlled clinical study.

Department of health and human services, food and drug administration, Hyalgan FDA 950027, 28 May 1997 relates to Hyalgan.

### SUMMARY OF THE INVENTION

The present invention is provides compositions comprising hyaluronic acid for treating sprain and/or strain in a animal or human in need of such treatment. The compositions comprise a therapeutically effective amount of hyaluronic acid for administration around the soft tissue to be treated. Soft - tissue may be selected from, but not limited to, muscle, fascia, tendon and ligament. In aspects, the method comprises an administration mode selected from the group consisting of periarticular, peri-ligamentous, peri-fascial and peri-musculotendinous administration.

It is now demonstrated that the treatment of soft tissue injury such as sprains and strains can be done using HA around (i.e. "peri") the injured soft tissue. This is in contrast to intra-articular administration of HA as previously described for other clinical indications. In aspects where the tissue is ligamentous or musculo-tendinous, the peri-articular, peri-ligamentous, peri-fascial or peri-musculo-tendinous administration of HA to the injured or susceptible ligamentous or musculo-tendinous tissue creates an internal scaffold or biocompatible internal support (i.e. an internal brace or scaffold) which provides structure and stability of form and function to the injured tissue thus allowing healing of the injured tissue to occur. This type of support provides protection to the site of injury by restricting further undesired or injurious movement at the site, and/or preventing further re-injury while healing is allowed to occur. The method of the invention is relatively simple to prepare and administer. The HA remains stable over time and be effective at dose regimens that are associated with minimal toxicity.

The compositions of the invention for treatment of an animal or human with a sprain or strain using purified HA of from about 0.3 to 3000 kDa molecular weight (any ranges there between) in an amount effective to treat the sprain and/or strain in the animal or the human.

In aspects, the HA may be provided as a composition comprising a suitable pharmaceutical carrier as is understood by one of skill in the art.

According to an aspect of the present invention is a composition comprising hyaluronic acid for treating injured soft tissue characterized by sprain and/or strain of such tissue, the composition comprising HA for administration to said injured soft tissue, wherein said administration is peri-articular, peri-ligamentous, peri-fascial or peri-musculotendinous.

According to another aspect of the present invention is the composition comprising hyaluronic acid for peri-articular, peri-fascial, peri-ligamentous and/or peri-musculotendinous use for the treatment of sprained and/or strained soft tissue. Soft tissues may include but not be limited to muscle, fascia, tendon and ligament.

According to another aspect of the present invention is the composition comprising hyaluronic acid for peri-articular, peri-fascial, peri-ligamentous and/or peri-musculotendinous use of HA for the treatment of shin splints.

According to an aspect of the present invention is a compositon comprising hyaluronic acid for effectively treating ligamentous sprain or musculo-tendinous strain in an animal, including a human. In aspects, the HA is for administration by periarticular (peri-ligamentous, peri-fascial and/or peri-musculotendinous) administration.

Still another aspect of the present invention is to provide a composition for treating sprain or strain that is minimally toxic to the recipient.

Yet another aspect of the present invention is to provide a composition to form an inner brace providing structure, stability of form and function, and allowing healing to occur in structures including, but not limited to, ligaments, tendons and muscles.

Another object of the present invention is to provide a composition to form a scaffold or internal support (i.e. internal brace or scaffold) providing structure, stability of form and function, and allowing healing of the strained and/or strained tissue to occur following periarticular (peri-ligamentous, peri-fascial and/or peri-musculotendinous) administration of HA.

Another aspect of the present invention is to provide a composition that potentiates the concomitant treatment of sprain or strain in an animal, including a human. In aspects such treatment may include but not be limited to the use of NSAIDs (such as but not limited to aspirin, ibuprofen and naproxen), cyclooxygenase-2 inhibitors, corticosteroids and the RICE method.

Still another aspect of the present invention is to provide a composition that potentiates rehabilitation of sprain or strain.

Another aspect of the present invention is to provide a composition that potentiates physical treatments of sprain or strain.

Yet another aspect of the present invention is to provide a composition that potentiates the healing effect of heat or cold in the treatment of sprain or strain.

Still another aspect of the present invention is to provide a composition that potentiates the healing effect of ultrasound in the treatment of sprain or strain.

Another aspect of the present invention is to provide a composition that potentiates the healing effect of electrical stimulation in the treatment of sprain or strain.

Still another aspect of the present invention is to provide a composition that potentiates the healing effect of compression in the treatment of sprain or strain.

Yet another aspect of the present invention is to provide a composition that potentiates the healing effect of elevation in the treatment of sprain or strain.

Another aspect of the present invention is to provide a composition that potentiates the healing effect of immobilization in the treatment of sprain or strain, wherein the immobilization includes, but is not limited to, a brace or a plaster cast.

Yet another aspect of the present invention is to provide a composition that potentiates the healing effect of surgery in the treatment of sprain or strain.

These and other objects, features and advantages of the present invention will become apparent after a review of the following detailed description of the disclosed embodiment and the appended claims.

### DETAILED DESCRIPTION OF THE INVENTION

The present invention is a novel composition for treating injured soft tissue such as muscle, fascia, tendons and ligaments. Injury such as sprain, strain or shin splint is treated in accordance with the invention by the administration of HA having a molecular weight of from about 0.3 to 3000 kDa in a therapeutically effective amount to alleviate the injury. The injury may be acute or chronic. The HA forms a scaffold providing structure, stability of form and function, and allows healing of the sprain and/or strain to occur following administration. In aspects, the injury may be a shin splint where the anterior tibial muscle is torn away from the bone. The HA may be used alone or as a pharmaceutical composition comprising a pharmaceutically acceptable carrier.

The method of the invention may be used concomitantly with known treatments for sprain and/or strain commonly used by one of ordinary skill in the art. Such treatments include, but are not limited to, the use of nonsteroidal anti-inflammatory drugs (NSAIDs), RICEs, plaster casts, braces, cyclooxygenase-2 inhibitors, corticosteroids, rehabilitation, physical therapies, heat and/or cold treatments, ultrasound, electrical treatments, surgery and combinations thereof.

The HA is administered to the outside (i.e. around, "peri") of the injured soft tissue, in this manner it may be administered peri-articularly, peri-ligamentously and/or peri-musculotendinously. Methods of making periarticular injections are known to one of ordinary skill in the art. Such injections are generally subcutaneous and target the vicinity of a joint, especially near the insertions or origins of muscle tendons and ligaments (peri-ligamentous and/or peri-musculotendinous). While not wanting to be bound by the following statements, it is believed that the HA infiltrates around and between the tissues, at their interface, thereby supporting these tissues in a scaffold and acting as a form of an internal brace that helps during the healing process.

As used herein "hyaluronic acid (HA)" includes but is not limited to hyaluronan, hyaluronate, salts of HA, homologues, analogues, derivatives, complexes, esters, fragments, and subunits of HA that are effective to form an internal brace providing structure, stability of form and function, and allowing healing to occur following periarticular administration and to potentiate the activity of other therapeutic agents used for sprain and/or strain.

The HA for use in the present invention may be any variety of forms having different molecular weights as is understood by one of skill in the art. In aspects, the HA may have an average molecular weight of about 30-750 kDa, about 50-750 kDa, about 500-750 kDa, about 30 to more than 750 kDa, or more than about 750 kDa. These forms of HA may be free of species of HA below about 30 kDa. Other forms of HA that may be used in the present invention include those forms having an average molecular weight of about 30-750 kDa, about 50-750 kDa, about 500-750 kDa, about 30 to more than 750 kDa, or more than about 750 kDa, a nd also include HA of molecular weight below about 30 kDa. In one embodiment, HA that may be used in the present invention has an average molecular weight of about 30-750 kDa, about 50-750 kDa, about 500-750 kDa, about 30 to more than 750 kDa, or more than about 750 kDa and also includes species of HA that are about 24 kDa.

In one aspect of the invention, the form of HA that may be used in the present invention has an average molecular weight below 50 kDa, or below 30 kDa. In one embodiment, the HA that may be used in the present invention has an average molecular weight of about 24 kDa. In another embodiment, HA may be used with an average molecular weight between about 0.3 kDa and 30 kDa. In another embodiment, HA may be used with an average molecular weight between about 10 kDa and 30 kDa.

HA is highly viscous, electronegative and hydrophilic. Various methods for the isolation, purification, fractionation or modification of HA are known to those skilled in the art. HA in its many forms is also readily available from many vendors or manufacturers as is understood by one of skill in the art such as Bioniche Life Sciences Inc, Canada; Anika Therapeutics, USA; Chemedica, Switzerland; Fidia, Italy; Genzyme (Biomatrix), USA; Hyalogic, USA; Hyalose, USA; Lifecore, USA; Seigakaku, Japan; Società Prodotti Antibiotici, Italy and Tedec Meiji, Japan. Specific hyaluronan compositions are also available for example from the following suppliers: BioMatrix Inc. Ridgefield, N.J. (Synvisc.TM., a 90:10 mixture of a hylan fluid and hylan gel); Fidia S.p.A., Abano Terme, Italy (Hyalgan™, the sodium salt of a rooster comb-derived hyaluronic acid (about.500,000 to about 700,000 MW)); Kaken . Pharmaceutical Co., Ltd., Tokyo, Japan (Artz™, a 1% solution of a rooster-comb derived hyaluronic acid, about 700,000 MW); Pharmacia AB, Stockholm, Sweden (Healon™, a rooster-comb derived hyaluronic acid, about 4 x 10⁶ MW); Genzyme Corporation, Cambridge, Mass. (Surgicoat™, a recombinant hyaluronic acid); Pronova Biopolymer, Inc. Portsmouth, N.H. (Hyaluronic Acid FCH, a high molecular weight (e.g., about 1.5-2.2 x 10⁶ MW) hyaluronic acid prepared from cultures of Streptococcus zooepidemicus; Sodium Hyaluronate MV, about 1.0-1.6 x 10⁶ MW and Sodium Hyaluronate LV, about 1.5-2.2 x 10⁶ MW); Calbiochem-Novabiochem AB, Lautelfingen, Switzerland (Hyaluronic Acid, sodium salt (1997 company catalog number 385908) prepared from Streptococcus sp.); Intergen Company, Purchase, N.Y. (a rooster-comb derived hyaluronic acid, >1 x 10⁶ MW); Diosynth Inc., Chicago, Ill.; Amerchol Corp., Edison, N.J. and Kyowa Hakko Kogyo Co., Ltd., Tokyo, Japan.

As used herein, "structure" refers to structures associated with joints including, but not limited, to ligaments, fascia, tendons and muscles.

As used herein, the word "response" refers to improving sprains and/or strains by forming an internal scaffold providing structure following periarticular administration.

As used herein, "potentiates" relates to a degree of synergism that is greater than additive.

As used herein, "synergism" relates to the coordinated action of two or more agents.

The present invention provides a method comprising administration of HA around ("peri") damaged soft tissue. The HA may be provided alone or as a composition comprising HA and a pharmaceutically acceptable carrier, wherein the composition is administered periarticularly, peri-ligamentously, peri-fascially and/or peri-musculotendinously to an animal, including humans, having an injury such as a sprain, strain or shin splint in an amount effective to form an inner brace providing structure and to decrease and/or to eliminate symptoms associated with the injury. Administration of the HA compositions may be used alone or in conjuction with other therapeutic modalities used for the treatment of sprain and/or strain to potentiate their effect. Such agents and methods include, but are not limited to, anti-inflammatory drugs, NSAIDs, corticosteroids, inhibitors of cyclooxygenase-2, RICE method, physical treatment, rehabilitation, heat and/or cold treatment, ultrasound therapy, electrical treatment such as piezoelectric and other forms of transcutaneous electrical treatment commonly used to treat musculoskeletal and joint injuries, elevation, compression, immobilization, immobilization devices, braces, plaster casts and surgery.

The therapeutic effectiveness of HA may be increased by methods including, but not limited to, chemically supplementing the HA, complexing the HA to biological or chemical carriers or coupling HA to tissue-type or cell-type directed ligands or antibodies.

Administration of an effective amount of HA to an animal, including a human, is a therapeutic treatment that prevents, treats or eliminates an acute or chronic soft tissue condition including, but not limited to, sprain, strain and shin splints.

### Pharmaceutically Acceptable Carriers

The HA may be administrated alone or in a pharmaceutically carrier including, but not limited to, a liquid carrier, a solid carrier or both.

Liquid carriers are aqueous carriers, non-aqueous carriers or both and include, but are not limited to, aqueous suspensions, dimethyl sulfoxide, ethanol, oil emulsions, water in oil emulsions, water-in-oil-in-water emulsions, site-specific emulsions, long-residence emulsions, sticky-emulsions, microemulsions and nanoemulsions. Solid carriers are biological carriers, chemical carriers or both and include, but are not limited to, particles, microparticles, nanoparticles, microspheres, nanospheres, bacterial cell wall extracts and biodegradable or non-biodegradable natural or synthetic polymers. Methods used to complex HA to a solid carrier include, but are not limited to, direct adsorption to the surface of the solid carrier, covalent coupling to the surface of the solid carrier, either directly or via a linking moiety, and covalent coupling or electrostatic coupling to the polymer used to make the solid carrier. Optionally, HA can be stabilized by the addition of non-ionic or ionic polymers such as polyoxyethylenesorbitan monooleates (TWEENs).

Preferred aqueous carriers include, but are not limited to, water, saline and pharmaceutically acceptable buffers such as phosphate buffers. Preferred non-aqueous carriers include, but are not limited to, a mineral oil or a neutral oil including, but not limited to, a diglyceride, a triglyceride, a phospholipid, a lipid, an oil and mixtures thereof, wherein the oil contains an appropriate mix of polyunsaturated and saturated fatty acids. Examples include, but are not limited to, soybean oil, canola oil, palm oil, olive oil and myglyol, wherein the fatty acids can be saturated or unsaturated. Optionally, excipients may be included regardless of the pharmaceutically acceptable carrier used to present the sequence to the responding cells. These excipients include, but are not limited to, anti-oxidants, buffers, and bacteriostats, and may include suspending agents and thickening agents.

### Combination Therapy

HA may be administered alone, or in combination with other therapeutic modalities including, but not limited to, anti-inflammatory drugs, NSAIDs, corticosteroids, inhibitors of cyclooxygenase-2, RICE method, physical treatment, rehabilitation, heat and/or cold treatment, ultrasound therapy, electrical treatment, elevation, compression, immobilization, immobilization device, braces, plaster casts and surgery. These therapeutic agents such as anti-inflammatory drugs, NSAIDs, corticosteroids, and inhibitors of cyclooxygenase-2 are administered using dosages and routes known to one of ordinary skill in the art. For example, anti-inflammatory drugs, NSAIDs, corticosteroids, and inhibitors of cyclooxygenase-2 may be administered orally. Corticosteroids may also be administered intravenously, topically, into a joint or through other routes known to one of ordinary skill in the art of administering corticosteroids.

In accordance with the present invention, the route of administration of HA includes, but is not limited to, periarticular, peri-ligamentous, peri-fascial or peri-musculotendinous injection. Any suitable device such as a syringe may be used to administer the HA or HA composition as is known to one of skill in the art.

In aspects, the amount of HA administered per dose is from about 0.001 to 1000 mg, more specifically from about 0.1 to 100 mg, from about 1 to 10 mg, and from about 0.1 mg to 5 mg. The volume per dose may be about 0.01 to 5.0 ml per dose, and in aspects from about 0.1 to 2.0 ml per dose, about 0.5 to 1.0 ml per dose or 0.01 to 1.0 ml per dose. The concentration of the HA provided in the composition may be in the range of about 5 to 100 mg/ml of the solution, in aspects 5 to 50 mg/ml of the solution and any range therebetween. The administration usually occurs in the vicinity of the sprain, strain or shin splint.

The administration of HA or HA plus other therapeutic modalities, the amount per dose, the dose schedule and the method of administration should be decided by the practitioner using methods known to those skilled in the art and will depend on the type of disease, the severity of the disease, the location of the disease and other clinical factors such as the size, weight and physical condition of the recipient. As such, the HA may be administered once, twice or several times as directed by the physician in amounts and concentrations as directed by the physician. The HA or HA plus other therapeutic agents or methods can be administered or applied in a single dose treatment, in multiple dose treatments or continuously infused on a schedule and over a period of time appropriate to the disease being treated, the condition of the recipient and the route of administration. Moreover, the therapeutic agents or methods can be administered or applied before, at the same time as, or after administration of HA.

The following examples will serve to further illustrate the present invention without, at the same time, however, constituting any limitation thereof. On the contrary, it is to be clearly understood that resort may be had to various other embodiments, modifications, and equivalents thereof which, after reading the description herein, may suggest themselves to those skilled, in the art without departing from the spirit of the invention and/or the scope of the appended claims.

### EXAMPLE 1

### HA preparation

The HA may have an average molecular weight of 30-750 kDa, 50-750 kDa, 500-750 kDa, 30 to more than 750 kDa, or more than 750 kDa. These forms of HA may be free of species of HA below 30 kDa. Other forms of HA that may be used in the present invention include those forms having an average molecular weight of 30-750 kDa, 50-750 kDa, 500-750 kDa, 30 to more than 750 kDa, or more than 750 kDa, and also include HA of molecular weight below 30 kDa, including HA species of 0.3 kDa to 30 kDa, 10 kDa to 30 kDa or about 24 kDa. In one embodiment, HA that may be used in the present invention has an average molecular weight of 30-750 kDa, 50-750 kDa, 500-750 kDa, 30 to more than 750 kDa, or more than 750 kDa and also includes species of HA that are about 24 kDa.

Another form of HA that may be used in the present invention has an average molecular weight below 50 kDa, or below 30 kDa. In one embodiment, the HA that may be used in the present invention has an average molecular weight of about 24 kDa. In another embodiment, HA may be used with an average molecular weight between about 0.3 kDa and 30 kDa. In another embodiment, HA may be used with an average molecular weight between about 10 kDa and 30 kDa.

Vials of HA are conveniently stored at room temperature (10°-30°C)**.**

### EXAMPLE 2

### Efficacy of HA on ankle sprain following periarticular administration

The study was conducted in accordance with good clinical practice and in compliance with the requirement of the International Conference on Harmonization and Declaration of Helsinki. On Day 1 (within 48 hours of the injury), the patients were examined to assess whether they meet all the inclusion criteria and none of the exclusion criteria after signing the informed consent. The inclusion criteria were: 18 years and older, first or second degree lateral ankle sprain within 48 hours of ad ministration of study drug, reported moderate (45-60 mm) to severe (>60mm) ankle pain on full weight bearing on the Patient's Assessment of Ankle Pain using a 100 mm Visual Analogue Scale (VAS). This scale was available for the duration of the clinical follow-up. Patients with bilateral ankle sprain, ipsilateral knee injury, third-degree sprain, or previous ankle sprain within 6 months were excluded. Also excluded were patients who had recently used anti-inflammatory drugs, muscle relaxants, or certain classes of psychotropic medications that could confound the results. In addition, patients with sensitivity or allergy to NSAIDs or sulfonamides as well as patients with a history of serious GI, renal, or hepatic disease were excluded. Patients with other rheumatic diseases or a history of drug or alcohol abuse were also excluded.

After enrollment, patients received periarticular injections of HA (study 1) or an inhibitor of cyclooxygenase-2 or a NSAID (study 2). Periarticular administration of HA having an average molecular weight of 500-750 kDa was done on day 1 and on day 4 (+/- 1 day) by using a 25-27 gauge needle and a 3 cc syringe. Eight to ten mg of HA (0.8-1.0 ml of HA) was injected, without anesthetic (study 1). Oral treatments with an inhibitor of cyclooxygenase-2, celecoxib 200 mg BID, or with a NSAID, naproxen 500 mg BID, were done for 7 days (study 2).

Clinical assessments were performed at baseline, and then before treatment on Day 4 and Day 8 (end of study). The measures of efficacy were the following: 1) the Patient's Assessment of Ankle Pain VAS (100 mm visual analogue scale) on weight bearing; 2) the Patient's Global Assessment (grade 1 to 5; very poor to very good); 3) the Patient's Satisfaction Assessment (a 10 point scale); and, 4) the Physician's Satisfaction Assessment (a 10 point scale).

Results are shown in Table 1 and Table 2. In Table 1, 7 patients received HA. In Table 2, 199 patients received celecoxib and 198 patients received naproxen.

**Table 1**

| | | | | | | | | |
|---|---|---|---|---|---|---|---|---|
| Mean of Patient's Assessment of Ankle Pain VAS (100 mm visual analogue scale) on weight bearing, percentage of patients with increase in Patient's Global Assessment (improved by one or more grades), mean of Patient's Satisfaction Assessment (a 10 point scale) and mean of Physician's Satisfaction Assessment (a 10 point scale) for patients having received HA at Day 1 and Day 4. | | | | | | | | |

| Treatment | Mean VAS Weight-bearing (100 mm) | | Percentage increase in Patient's Global Assessment | | Mean Patient's Satisfaction Assessment (10 point scale) | | Mean Physician's Satisfaction Assessment (10 point scale) | |
|---|---|---|---|---|---|---|---|---|
| | At day 4 | At day 8 | At day 4 | At day 8 | At day 4 | At day 8 | At day 4 | At day 8 |
| Periarticular HA | 52.0 | 15.1 | 100% | 100% | 4.6 | 9.0 | 7.7 | 9.9 |

**Table 2**

| | | | | | | | | |
|---|---|---|---|---|---|---|---|---|
| Mean of Patient's Assessment of Ankle Pain VAS (100 mm visual analogue scale) on weight bearing, percentage of patients with increase in Patient's Global Assessment (improved by one or more grades), mean of Patient's Satisfaction Assessment (a 10 point scale) and mean of Physician's Satisfaction Assessment (a 10 point scale) for patients having received celecoxib or naproxen at Day 1 and Day 4. | | | | | | | | |

| Treatment | Mean VAS Weight-bearing (100 mm) | | Percentage increase in Patient's Global Assessment | | Mean Patient's Satisfaction Assessment (10 point scale) | | Mean Physician's Satisfaction Assessment (10 point scale) | |
|---|---|---|---|---|---|---|---|---|
| | At day 4 | At day 8 | At day 4 | At day 8 | At day 4 | At day 8 | At day 4 | At day 8 |
| Celecoxib | 31.9 | 15.0 | 71% | 89% | n.d. | 8.8 | n.d. | 8.7 |
| Naproxen | 29.0 | 15.3 | 72% | 90% | n.d. | 8.7 | n.d. | 8.6 |

Table 1 shows the efficacy of periarticular administration of HA to patients with ankle sprain. The efficacy of periarticular administration of HA was comparable to the efficacy obtained with celecoxib, an inhibitor of cyclooxygenase-2, and naproxen, an NSAID (Table 2).

### EXAMPLE 3

### Efficacy of HA having an average molecular weight of 500-750 kDa in Ilio-tibial band syndrome following periarticular (peri-musculo-tendinous) administration

The experiment was performed in 8 young and middle-aged adults who had Ilio-tibial band (ITB) syndrome symptoms of unilateral pain and stiffness at the proximal band end for at least 3 months (range 3-9 months) with negative studies of hip articular pathology (radiograph). Patients were administered 2.5 cc 10mg/ml HA with MW range 500-6800 kDa on presentation, and at days 7 and 14 post initial injection. Injections were administered HA using a 25 gauge needle and 3 cc syringe without anesthetic. Clinical assessments were done before treatment on days 1, 7 and 14 (+/- 2 days). The measures of efficacy included: 1) patient assessment of pain on weight bearing using 100mm VAS; 2) patient global assessment of effect (categorical scale 1-5); 3) patient satisfaction with injection procedure using a 10-point VAS; 4) ITB flexibility using a modified Ober's test (positive or negative). Results are shown in Table 3. Eight patients received HA and followup assessments A reduction in pain, an increase in patient global assessment of effect and satisfaction and improvement in Ober's test score at day 14.

**Table 3**

| | | | | | | | | |
|---|---|---|---|---|---|---|---|---|
| Mean of Patient's Assessment of ITB Pain VAS (100 mm visual analogue scale) on weight bearing, percentage of patients with increase in Patient's Global Assessment of effect (improved by one or more grades), mean of Patient's Satisfaction Assessment (a 10 point scale) and percentage of Ober's test scores change to negative for patients having received HA at Day 1 and Day 14. | | | | | | | | |

| Treatment | Mean VAS Weight-bearing (100 mm) | | Percentage increase in Patient's Global Assessment | | Mean Patient's Satisfaction Assessment (10 point scale) | | Percentage Ober's test improvement (change from positive to negative) | |
|---|---|---|---|---|---|---|---|---|
| | At day 1 | At day 14 | At day 1 | At day 14 | At day 1 | At day 14 | At day 1 | At day 14 |
| Peri-ITB HA | 61.0 | 21.3 | 0% | 80% | O | 8.7 | 0 | 100% |

### EXAMPLE 4

### Administration of HA for treatment of shin splints

Four patients presented with bilateral anterior shin pain were diagnosed with shin splints. Each received baseline assessment of pain on weight bearing with both legs and each leg separately (single leg stand). Pain was scored using a 100 mm VAS. Patients were asked to identify the area of greatest pain over 5cm. Patients were then injected peri-fascial in the region identified on one leg only. The treatment leg was randomly chosen. Patients received no other concomitant therapy. Patients received 3.0ml HA (HA as described in example 3) through a 27 gauge needle under sterile field conditions. Patients returned for repeat VAS assessment and the re-injection at the identified site on days 4 and 8. Results are shown in Table 4. All patients tolerated the procedure and completed assessments and injection series. Baseline VAS was similar in both and single legs. At follow up on day 8, VAS decreased in the treated leg more than the untreated and both legs.

**Table 4**

| | Baseline | Day 4 | Day 8 |
|---|---|---|---|
| Both legs (cm) | 74.3 | 71.9 | 67.7 |
| Treatment leg (cm) | 76.8 | 58.6 | 40.1 |
| Control leg (cm) | 77.9 | 68.2 | 74.7 |

### EXAMPLE 5

### Formation of an inner brace following periarticular administration of HA

The conjugation of [¹¹C] to HA is performed as described by Westerberg et al. Nucl. Med. Biol. 22:251-256, 1995. HA of 24 kDa, 500-750 kDa, 500-750 kDa containing HA of less than 30 kDa, HA of greater than about 750 kDa, HA of greater than about 750 kDa containing HA of less than 30 kDa, are separately used for conjugation. Twelve individuals with an ankle sprain are recruited for the experiment. Individuals are divided into 3 groups. Group 1 individuals receive periarticular [¹¹C]-HA of 24 kDa; Group 2 individuals receive periarticular [¹¹C]-HA of 500-750 kDa, Group 3 individuals receive periarticular [¹¹C]-HA of 500-750 kDa containing a substantial amount of [¹¹C]-HA of less than 30 kDa, Group 4 individuals receive periarticular [¹¹C]-HA of greater than about 750 kDa, Group 5 individuals receive periarticular [¹¹C]-HA of greater than about 750 kDa containing a substantial amount of [¹¹C]-HA of less than 30 kDa. Injection volumes of HA are about 0.8 to 1.0 ml. Positron Emission Tomography (PET) is used for assessment of structure and quantification of [¹¹C]-HA. Results show the formation of an inner brace following intra-articular administration of [¹¹C]-HA of 24 kDa, [¹¹C]-HA of 500-750 kDa, [¹¹C]-HA of greater than about 750 kDa, [¹¹C]-HA of 500-750 kDa containing a substantial amount of [¹¹C]-HA of less than 30 kDa, and [¹¹C]-HA of greater than about 750 kDa containing a substantial amount of [¹¹C]-HA of less than 30 kDa.

## Claims

1. A composition comprising hyaluronic acid (HA) for treating acutely or chronically injured soft tissue in an animal or human, wherein the composition comprises a therapeutically effective amount of HA for peri-articular, peri-ligamentous, peri-musculotendinous, and/or peri fascial administration around said injured soft tissue such that the HA creates an internal scaffold or biocompatible internal support that provides structure and stability to the injured soft tissue.

2. The composition of claim 1, wherein said injured soft tissue is selected from the group consisting of muscle**,** fascia, tendon and ligament.

3. The composition of claim 2, wherein said HA is provided as a composition together with a pharmaceutically acceptable carrier.

4. The composition of claim 3, wherein the HA has a molecular weight below about 30 kDa.

5. The composition of claim 3, wherein the HA has a molecular weight between about 10-3000 kDa.

6. The composition of claim 3, wherein the HA has a molecular weight between about 500-750 kDa.

7. The composition of claim 3, wherein the HA has a molecular weight between about 10-750 kDa.

8. The composition of claim 3, wherein the HA has molecular weight of greater than about 750 kDa.

9. The composition of claim 3, wherein said HA is provided with a combination of molecular weights.

10. The composition of claim 9, wherein the HA comprises a molecular weight between about 500-750 kDa and less than about 30 kDa.

11. The composition of claim 9, wherein the HA comprises a molecular weight of greater than about 750 kDa and less than about 30 kDa.

12. The composition of claim 3, wherein said HA is for administration in amount of from about 0.001 to about 1000mg per dose.

13. The composition of claim 12, wherein said HA is for administration in amount of from about 0.1 to about 100mg per dose.

14. The composition of claim 13, wherein said HA is for administration in amount of from about 1 to about 10mg per dose.

15. The composition of claim 3, wherein said HA is provided in a volume dose of about 0.01 to 5.0 ml.

16. The composition of claim 15, wherein said HA is provided in a volume dose of about 0.01 to 2.0 ml.

17. The composition of claim 16, wherein said HA is provided in a volume dose of about 0.5 to 1.0 ml.

18. The composition of claim 17, wherein said HA is provided in a volume dose of about 0.01 to 1.0 ml.

19. The composition of claim 3, wherein said HA is provided in an amount of about 5 to 100mg/ml.

20. The composition of any one of claims 1 to 19, wherein said composition is for administration once or several times.

21. The composition of claims 1 or 2, wherein said injured soft tissue is as a result of a sprain, strain or shin splint.

22. The composition of claim 21, wherein said injured soft tissue is as a result of a sprain.

23. The composition of claim 21, wherein said injured soft tissue is as a result of a strain.

24. The composition of claim 21, wherein said injured soft tissue is as a result of a shin splint.

25. The composition of any one of claims 1 to 24, wherein said composition is for use in conjunction with one or more of an NSAID, a corticosteroid, an inhibitor of cyclooxygenase-2, RICE treatment, rehabilitation, physical treatment, electrical treatment, heat, cold, ultrasound, compression, elevation, immobilization, brace, or a plaster cast.

26. Use of a composition comprising hyaluronic acid in the manufacture of a medicament for treatment of acutely or chronically injured soft tissue in an animal or human, wherein the composition comprises a therapeutically effective amount of HA for peri-articular, peri-ligamentous, peri-musculotendinous, and/or peri-fascial administration, around said injured soft tissue such that the HA creates an internal scaffold or biocompatible internal support that provides structure and stability to the injured soft tissue.

## Patentansprüche

1. Zusammensetzung, welche Hyaluronsäure (HA) umfaßt, zur Behandlung von akut oder chronisch verletztem Weichgewebe in einem Tier oder einem Menschen, wobei die Zusammensetzung eine therapeutisch wirksame Menge an HA für die periartikuläre, periligamentäre, perimuskulotendinöse und/oder perifasziale Verabreichung um das verletzte Weichgewebe herum umfaßt, so daß die HA ein inneres Gerüst oder eine biokompatible innere Abstützung erzeugt, das bzw. die dem verletzten Weichgewebe Struktur und Stabilität verleiht.

2. Zusammensetzung nach Anspruch 1, wobei das verletzte Weichgewebe aus der Gruppe ausgewählt ist, bestehend aus Muskeln, Faszien, Sehnen und Bändern.

3. Zusammensetzung nach Anspruch 2, wobei die HA als eine Zusammensetzung zusammen mit einem pharmazeutisch verträglichen Träger bereitgestellt wird.

4. Zusammensetzung nach Anspruch 3, wobei die HA ein Molekulargewicht unterhalb von etwa 30 kDa hat.

5. Zusammensetzung nach Anspruch 3, wobei die HA ein Molekulargewicht von zwischen etwa 10-3000 kDa hat.

6. Zusammensetzung nach Anspruch 3, wobei die HA ein Molekulargewicht von zwischen etwa 500-750 kDa hat.

7. Zusammensetzung nach Anspruch 3, wobei die HA ein Molekulargewicht von zwischen etwa 10-750 kDa hat.

8. Zusammensetzung nach Anspruch 3, wobei die HA ein Molekulargewicht von mehr als etwa 750 kDa hat.

9. Zusammensetzung nach Anspruch 3, wobei die HA mit einer Kombination von Molekulargewichten bereitgestellt wird.

10. Zusammensetzung nach Anspruch 9, wobei die HA ein Molekulargewicht von zwischen etwa 500-750 kDa und weniger als etwa 30 kDa umfaßt.

11. Zusammensetzung nach Anspruch 9, wobei die HA ein Molekulargewicht von mehr als etwa 750 kDa und weniger als etwa 30 kDa umfaßt.

12. Zusammensetzung nach Anspruch 3, wobei die HA für eine Verabreichung in einer Menge von etwa 0,001 bis etwa 1000 mg pro Dosis bestimmt ist.

13. Zusammensetzung nach Anspruch 12, wobei die HA für eine Verabreichung in einer Menge von etwa 0,1 bis etwa 100 mg pro Dosis bestimmt ist.

14. Zusammensetzung nach Anspruch 13, wobei die HA für eine Verabreichung in einer Menge von etwa 1 bis etwa 10 mg pro Dosis bestimmt ist.

15. Zusammensetzung nach Anspruch 3, wobei die HA in einer Volumendosis von etwa 0,01 bis 5,0 ml bereitgestellt wird.

16. Zusammensetzung nach Anspruch 15, wobei die HA in einer Volumendosis von etwa 0,01 bis 2,0 ml bereitgestellt wird.

17. Zusammensetzung nach Anspruch 16, wobei die HA in einer Volumendosis von etwa 0,5 bis 1,0 ml bereitgestellt wird.

18. Zusammensetzung nach Anspruch 17, wobei die HA in einer Volumendosis von etwa 0,01 bis 1,0 ml bereitgestellt wird.

19. Zusammensetzung nach Anspruch 3, wobei die HA in einer Menge von etwa 5 bis 100 mg/ml bereitgestellt wird.

20. Zusammensetzung nach einem der Ansprüche 1 bis 19, wobei die Zusammensetzung für die einmalige oder mehrmalige Verabreichung bestimmt ist.

21. Zusammensetzung nach den Ansprüchen 1 oder 2, wobei das verletzte Weichgewebe die Folge einer Verstauchung, einer Zerrung oder eines Kompartmentsyndroms ist.

22. Zusammensetzung nach Anspruch 21, wobei das verletzte Weichgewebe die Folge einer Verstauchung ist.

23. Zusammensetzung nach Anspruch 21, wobei das verletzte Weichgewebe die Folge einer Zerrung ist.

24. Zusammensetzung nach Anspruch 21, wobei das verletzte Weichgewebe die Folge eines Kompartmentsyndroms ist.

25. Zusammensetzung nach einem der Ansprüche 1 bis 24, wobei die Zusammensetzung für die Verwendung zusammen mit einem oder mehreren von einem NSAID, einem Corticosteroid, einem Inhibitor von Cyclooxygenase-2, RICE-Behandlung, Rehabilitation, physikalischer Behandlung, elektrischer Behandlung, Wärme, Kälte, Ultraschall, Kompression, Elevation, Immobilisierung, Stützverband oder Gipsverband bestimmt ist.

26. Verwendung einer Zusammensetzung, welche Hyaluronsäure umfaßt, bei der Herstellung eines Medikaments zur Behandlung von akut oder chronisch verletztem Weichgewebe in einem Tier oder einem Menschen, wobei die Zusammensetzung eine therapeutisch wirksame Menge an HA für die periartikuläre, periligamentäre, perimuskulotendinöse und/oder perifasziale Verabreichung um das verletzte Weichgewebe herum umfaßt, so daß die HA ein inneres Gerüst oder eine biokompatible innere Abstützung erzeugt, das bzw. die dem verletzten Weichgewebe Struktur und Stabilität verleiht.

## Revendications

1. Composition comprenant de l'acide hyaluronique (HA) pour le traitement d'un tissu mou lésé de manière aiguë ou chronique chez un animal ou un être humain, ladite composition comprenant une quantité thérapeutiquement efficace de HA pour l'administration périarticulaire, périligamenteuse, périmusculotendineuse et/ou périfaciale, autour dudit tissu mou lésé, de telle sorte que le HA crée un échafaudage interne ou un support interne biocompatible qui confère une structure et une stabilité au tissu mou lésé.

2. Composition suivant la revendication 1, dans laquelle ledit tissu mou lésé est choisi dans le groupe consistant en un muscle, un fascia, un tendon et un ligament.

3. Composition suivant la revendication 2, dans laquelle ledit HA est fourni sous forme d'une composition en association avec un support pharmaceutiquement acceptable.

4. Composition suivant la revendication 3, dans laquelle le HA a un poids moléculaire inférieur à environ 30 kDa.

5. Composition suivant la revendication 3, dans laquelle le HA a un poids moléculaire d'environ 10 à 3000 kDa.

6. Composition suivant la revendication 3, dans laquelle le HA a un poids moléculaire d'environ 500 à 750 kDa.

7. Composition suivant la revendication 3, dans laquelle le HA a un poids moléculaire d'environ 10 à 750 kDa.

8. Composition suivant la revendication 3, dans laquelle le HA a un poids moléculaire supérieur à environ 750 kDa.

9. Composition suivant la revendication 3, dans laquelle ledit HA est fourni avec une association de poids moléculaires.

10. Composition suivant la revendication 9, dans laquelle le HA comprend un poids moléculaire d'environ 500 à 750 kDa et un poids moléculaire inférieur à environ 30 kDa.

11. Composition suivant la revendication 9, dans laquelle le HA comprend un poids moléculaire supérieur à environ 750 kDa et inférieur à environ 30 kDa.

12. Composition suivant la revendication 3, dans laquelle ledit HA est destiné à l'administration en une quantité d'environ 0,001 à environ 1000 mg par dose.

13. Composition suivant la revendication 12, dans laquelle ledit HA est destiné à l'administration en une quantité d'environ 0,1 à environ 100 mg par dose.

14. Composition suivant la revendication 13, dans laquelle ledit HA est destiné à l'administration en une quantité d'environ 1 à environ 10 mg par dose.

15. Composition suivant la revendication 3, dans laquelle ledit HA est fourni à une dose d'un volume d'environ 0,01 à 5,0 ml.

16. Composition suivant la revendication 15, dans laquelle ledit HA est fourni à une dose d'un volume d'environ 0,01 à 2,0 ml.

17. Composition suivant la revendication 16, dans laquelle ledit HA est fourni à une dose d'un volume d'environ 0,5 à 1,0 ml.

18. Composition suivant la revendication 17, dans laquelle ledit HA est fourni à une dose d'un volume d'environ 0,01 à 1,0 ml.

19. Composition suivant la revendication 3, dans laquelle ledit HA est fourni en une quantité d'environ 5 à 100 mg/ml.

20. Composition suivant l'une quelconque des revendications 1 à 19, ladite composition étant destinée à l'administration une ou plusieurs fois par jour.

21. Composition suivant la revendication 1 ou 2, dans laquelle ledit tissu mou lésé est dû à une entorse, une foulure ou à la présence d'une attelle tibiale.

22. Composition suivant la revendication 21, dans laquelle ledit tissu mou lésé est dû à une entorse.

23. Composition suivant la revendication 21, dans laquelle ledit tissu mou lésé est dû à une foulure.

24. Composition suivant la revendication 21, dans laquelle ledit tissu mou lésé est dû à la présence d'une attelle tibiale.

25. Composition suivant l'une quelconque des revendications 1 à 24, ladite composition étant destinée à être utilisée conjointement avec un ou plusieurs moyens consistant en un médicament anti-inflammatoire non stéroïdien (MAINS), un corticostéroïde, un inhibiteur de cyclooxygénase-2, un traitement RICE, une réhabilitation, un traitement physique, un traitement électrique, la chaleur, le froid, des ultrasons, une compression, une élévation, une immobilisation, une attelle ou un plâtre.

26. Utilisation d'une composition comprenant de l'acide hyaluronique dans la production d'un médicament destiné au traitement d'un tissu mou lésé de manière aiguë ou chronique chez un animal ou un être humain, dans laquelle la composition comprend une quantité thérapeutiquement efficace de HA pour l'administration périarticulaire, périligamenteuse, périmusculotendineuse et/ou périfaciale, autour dudit tissu mou lésé, de telle sorte que le HA crée un échafaudage interne ou un support interne biocompatible qui confère une structure et une stabilité au tissu mou lésé.
